# EUROPEAN PATENT APPLICATION

(11) **EP 4 785 891 A1**
(43) Date of publication of application: **05.08.2026**
(21) Application number: 25155166.9
(22) Date of filing: 31.01.2025
(51) Int. Cl.: A61B 34/20, A61B 17/34, A61B 90/13, A61B 90/00, A61B 17/00, A61B 34/10

(54) **COMPUTER VISION FOR 3D NEEDLE GUIDANCE IN MEDICAL PROCEDURES**

(71) Applicant: Lesage, Frédéric, 18000 centre-Val de Loire bourges (FR)
(72) Inventor: Lesage, Frédéric, 18000 centre-Val de Loire bourges (FR)

(57) **Abstract**

This invention introduces a real-time, three-dimensional (3D) guidance system for medical instruments, designed to improve accuracy, safety, and accessibility in minimally invasive procedures. The system leverages advanced computer vision technology and integrates multiple innovative components, including:
• **3D Camera System:** Aligns a virtual hologram of the patient's anatomy with their physical body for precise targeting.
• **Laser-Guided Medical Handle:** Equipped with an inertial measurement unit (IMU) and contact detection to define entry points and monitor needle alignment.
• **Dynamic Hologram Adjustment:** Continuously aligns the virtual hologram with real-time patient movements, ensuring accuracy during procedures.
• **Trajectory Guidance:** Displays real-time feedback on entry points, needle progression, and alignment with the target area.
• **Safety Features:** Includes breathing synchronization, collision avoidance, and alerts to minimize risks during interventions.
• **Portability:** Lightweight and portable design suitable for clinical, remote, or resource-limited settings.

This system enables precise and safe procedures such as biopsies, injections, and ablations while reducing dependency on highly specialized expertise. By democratizing access to advanced medical techniques, it enhances procedural outcomes and broadens healthcare accessibility globally.

## Description

### FIELD OF THE INVENTION

The present invention relates to the field of medical imaging and intervention systems, with a specific focus on needle guidance for minimally invasive procedures. This invention employs advanced computer vision techniques, including three-dimensional (3D) imaging, to monitor a patient's anatomy in real-time and guide medical instruments with precision. Utilizing a 3D camera, the system provides continuous spatial awareness and trajectory assistance, ensuring accurate and safe navigation during procedures such as biopsies, injections, and targeted ablations. The technology aims to enhance procedural accuracy, reduce complications, and improve outcomes through intuitive, real-time visual feedback for clinicians.

### BACKGROUND OF THE INVENTION

The integration of computer vision technology into Augmented Reality (AR) or Virtual Reality (VR) environments for medical procedures has attracted significant interest, thanks to its potential to improve precision and reduce risks in interventions such as biopsies, injections, and targeted ablations. While traditional guidance systems, including electromagnetic tracking and infrared optical tracking, have demonstrated their utility, they are frequently limited by significant challenges that compromise both reliability and ergonomics.

Electromagnetic tracking systems, while effective, are highly susceptible to environmental interference from nearby metallic objects or electromagnetic radiation emitted by medical equipment such as MRI machines or surgical tools. These interferences can cause inaccurate positioning and unreliable tracking, undermining the precision required for medical procedures. Additionally, electromagnetic systems often rely on bulky setups, including separate emitters and receptors, which can inadvertently be moved, leading to calibration errors.

Infrared (IR) tracking, although effective in certain contexts, has significant limitations. One key drawback is its reliance on an IR light source, typically a large and cumbersome lamp, which is difficult to transport between rooms, reducing its practicality in medical environments. Moreover, both IR tracking and electromagnetic tracking are incapable of detecting the patient's body. They lack the ability to recognize anatomical structures or accurately capture the patient's dimensions, making them unsuitable for procedures that demand precise overlay, accurate guidance, and safe navigation around critical anatomical features.

To overcome these limitations, the invention described here harnesses the precision of a 3D camera to enhance needle guidance in medical procedures. By leveraging advanced computer vision technology, the system automatically overlays a holographic representation of the patient's anatomy onto their physical body. This hologram provides detailed information about the procedural area, enabling clinicians to visualize critical structures and planned trajectories seamlessly within their field of view.

The system ensures the overlay's accuracy through a fitness measurement algorithm, which continuously evaluates and refines the alignment between the hologram and the patient's body in real-time. This dynamic adjustment guarantees precise and reliable alignment throughout the procedure, significantly enhancing both safety and procedural effectiveness.

The system facilitates the democratization of medical procedures by automating the primary steps involved, which will be detailed further in subsequent sections. By reducing the complexity of these steps, the system ensures that advanced medical interventions are no longer limited to specialists or constrained by traditional clinical settings. Its design emphasizes accessibility and simplicity, allowing healthcare providers at various levels of expertise to achieve high precision and effectiveness during procedures.

A key feature of the system is its lightweight and portable design, which eliminates the need for a fixed, dedicated operating room. Unlike conventional medical equipment that often requires significant infrastructure and setup, this system can be easily transported, making it highly adaptable for various environments. It extends the reach of advanced medical care to areas where access to fully equipped hospitals may be limited or non-existent. This portability also makes the system particularly suitable for home-based care, enabling interventions to be performed directly at the patient's residence. Such flexibility is invaluable for patients with mobility issues or those residing in remote locations, significantly improving their access to critical medical procedures.

Moreover, the system's user-friendliness is a transformative aspect. The instructions necessary to perform the procedure are preprocessed and integrated into the hologram, providing clear, step-by-step guidance throughout the intervention. This means that even non-specialist operators, including general practitioners or healthcare aides, can carry out the procedure with confidence and precision. The hologram acts as a visual tutor, guiding the operator through the process while ensuring adherence to medical protocols. This not only broadens the pool of potential users but also reduces the dependency on highly trained specialists, making advanced medical procedures more inclusive and scalable.

By combining portability, automation, and intuitive operation, the system represents a significant step forward in making advanced medical technologies accessible to a wider population, bridging gaps in healthcare delivery, and addressing critical needs in both urban and rural settings.

### SUMMARY OF THE INVENTION

The present invention introduces a system and method for real-time, three-dimensional guidance of medical instruments, such as a needle (23), utilizing virtual reality (VR) enhanced by laser assistance. This innovation overcomes the limitations of traditional tracking systems by delivering highly precise spatial tracking in complex medical environments. The system leverages advanced computer vision technology to align (C2) a virtual representation of the patient (38) with their physical body (10), enabling accurate guidance of the needle (23) to a specified target (11).

The guidance is achieved through various techniques detailed in this document. The system comprises two primary components: a stereo camera (31) integrated with a computer unit (33) that generates the virtual representation, and a medical handle (21) designed to accommodate different types of needles (23). The medical handle incorporates several subsystems, including a wireless emitter, an IMU, and a Laser LED (22). These components transmit data to the computer unit (33) to facilitate the operation (steps C3, C4, and C5).

The method includes the following steps:
**Step A: Image Acquisition of the Real Body**
   **1. Step A1: Acquire images using a medical imaging device, such as an MRI scanner or other modalities, to capture detailed internal body structures.**
   **2. Step A2: Send the acquired medical images to a database for further processing.**
**Step B: Creation of the Hologram**
   **1. Step B1: Load the patient's medical images into the system.**
   **2. Step B2: Perform automatic segmentation of skin, bones, and organs from the imaging data.**
   **3. Step B3: Identify specific targets within the body, such as areas for intervention or anatomical features.**
   **4. Step B4: Generate a holographic representation of the patient based on the segmented data and identified targets.**
   **5. Step B5: Push the generated hologram to a server for access and integration with the guidance system.**
**Step C: Medical Gesture**
   **1. Step C1: Load the hologram of the patient into the system.**
   **2. Step C2: Align the hologram with the patient's real body for precise overlay and guidance.**
   **3. Step C3: Use a laser to define the entry point for the medical instrument.**
   **4. Step C4: Determine the optimal angle for accessing the target while avoiding critical structures.**
   **5. Step C5: Operate on the patient using the holographic and laser-guided system for precise intervention.**

This system is particularly valuable in medical applications, such as biopsies, injections, and targeted treatments, where precision is critical. By reducing reliance on complex external tracking systems and providing enhanced safety and accuracy, the invention establishes a new standard for instrument guidance in VR-assisted interventions.

### BRIEF DESCRIPTION OF THE DRAWINGS

The following sections provide a more detailed explanation of the invention, accompanied by the attached drawings for reference.

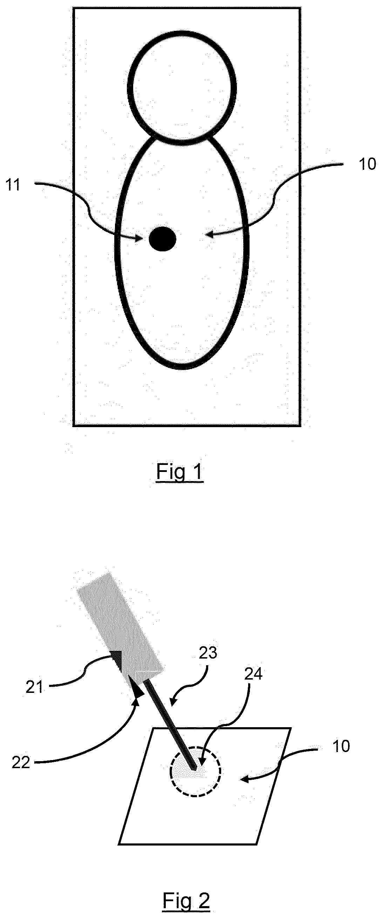

**[****Fig. 1]** Figure 1 illustrates a side view of a real patient (10) presenting with a pathology (11) requiring surgical intervention.
**[****Fig. 2]** Figure 2 illustrates the medical handle (21), which securely holds the needle (23) and incorporates a laser LED (22) to illuminate the entry point (24) on the patient's body (10).

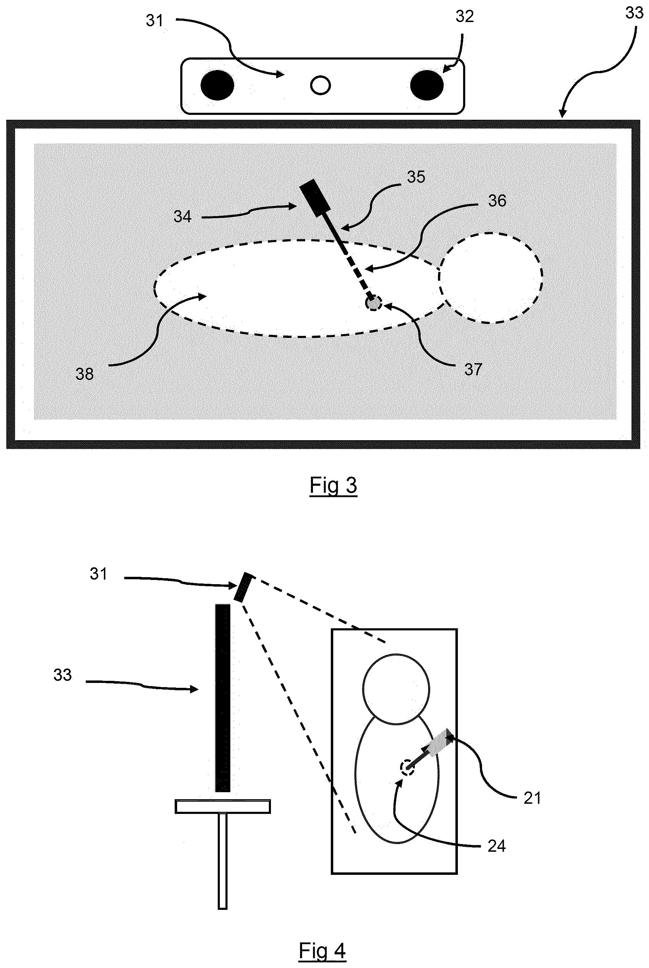

**[****Fig. 3]** Figure 3 depicts the computer unit (33), represented by a large screen, with a 3D camera system (31) equipped with multiple lenses (32) mounted on top. The screen displays a virtual representation (38) of the patient, highlighting the target area (37) to be reached. The user can also visualize the virtual medical handle (34), the virtual needle (35), and the planned trajectory (36).
**[****Fig. 4]** Figure 4 illustrates the scene during the medical procedure. The practitioner utilizes the medical handle (21) to guide the needle toward the entry point (24) while observing their movements on the screen (33), which is equipped with a 3D camera system (31) mounted on top.

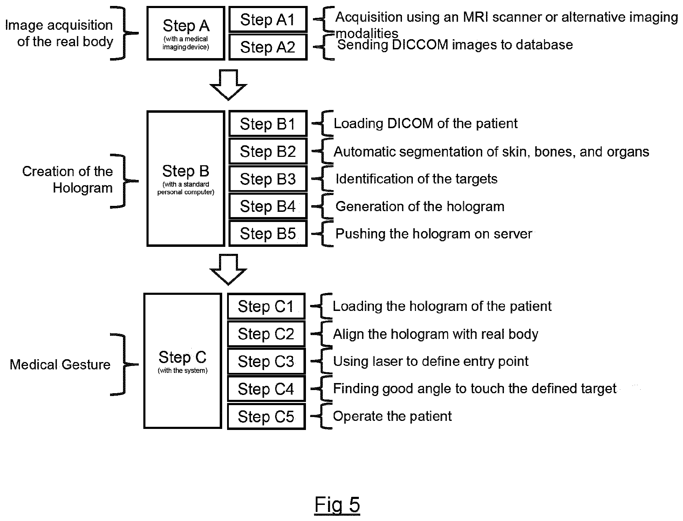

**[****Fig. 5]** Figure 5 presents a block diagram outlining a method for the real-time, three-dimensional guidance of a medical instrument, as detailed in the invention.

### DETAILED DESCRIPTION OF THE INVENTION

### Part 1: Core principles and design

The invention introduces an advanced system for guiding medical needles using virtual reality (VR) to support practitioners with precision and ease. This innovative approach overcomes the limitations of traditional tracking methods, such as susceptibility to electromagnetic interference, by incorporating a robust and interference-free tracking mechanism.

The system utilizes a 3D camera (31) to align a holographic representation of the patient (38) with their physical body (10) and continuously monitor any patient movement to ensure stability during the procedure. Additionally, the 3D camera (31) detects the laser LED (22) light to precisely identify the entry point for the needle. Equipped with a wireless transmitter, an inertial measurement unit (IMU), and contact detection modules integrated into the medical handle (21), the system can determine the exact point and moment the needle (23) touches the patient's body (10). By tracking the needle's angle and integrating all this data, the system computes a virtual trajectory (36) displayed on the screen (33). This trajectory enables the system to guide the practitioner in aligning the needle (23) precisely with the target (11), ensuring accurate and safe needle placement.

### Part 2: System Components

The system is composed of two main units: the first includes the 3D camera (31) and its attached control computer (33), while the second is the medical handle (21), designed to securely hold the needle (23).

### 1. 3D camera and control computer

The 3D camera (31) is managed by the control computer (33), which communicates wirelessly with the medical handle (21). The camera scans its field of view with sufficient precision to enable the control computer to align the captured volume with the hologram (38).

The control computer (33) processes the video stream from the 3D camera (31), applying smoothing techniques to reduce noise and instability. Its primary function is to provide the practitioner with a clear and accurate virtual representation of the key elements essential to guiding the medical procedure.

### 2. Medical handle

The medical handle (21) is primarily a versatile attachment system designed to accommodate needles of various sizes. The farther the target is from the skin, the longer the needle needs to be.

The key distinction of this system compared to other medical tracking technologies, such as electromagnetic or optical systems, lies in its design philosophy. Unlike these traditional systems, it is not intended to track the medical handle in free flight (i.e., when it is not in contact with the patient's body). While infrared (IR) or electromagnetic tracking systems can effectively track the medical handle both in contact with the patient and when suspended above, this capability is irrelevant for the procedure. What truly matters is the precise tracking of the needle (23) at the moment it touches the patient, which is the system's primary focus. What truly matters is the system's ability to accurately determine the exact location of the needle (23) when it is in contact with the patient (10), along with its angle. With these two critical pieces of information, the system can guide the practitioner on whether the needle is properly aligned to reach the target (11) upon penetrating the skin. This primary focus has been the driving force behind the design of the medical handle.

To fulfil its purpose, the medical handle (21) is equipped with several modules. A capacitive touch sensor detects when the needle (23) makes contact with the patient's body (10). At that precise moment, the medical handle (21) activates a laser to illuminate the entry point (24), providing the control computer (33) with the exact location where the needle touches the body. Before this point of contact, the control computer (33) has no knowledge of the medical handle's position.

Once contact is established, an inertial measurement unit (IMU) supplies angular data, which is transmitted to the control computer (33). Using these two pieces of information-position and angle-the system calculates the needle's trajectory (36).

Optionally, an integrated telemetry module located on the edge of the medical handle can provide additional information to the practitioner, such as the distance between the entry point and the medical handle. By subtracting this value from the needle's total length, the system can calculate the depth of the needle within the patient's body (10) and the remaining distance to the target (11).

### Part 3: Operational Methodology and Tracking Workflow

The operational methodology of the invention leverages virtual reality to provide precise and intuitive guidance for needle placement. Below is a step-by-step outline of the workflow, from initialization to real-time guidance:

### STEP A: Image Acquisition of the Real Body

Step A involves acquiring medical images using a dedicated acquisition system (A1), which will not be discussed in detail in this document. At the conclusion of this process, the images are expected to have high-quality edge definition of the patient's surface (skin), as this will be critical for aligning the future hologram (38) with the real body (10). Additionally, the images should clearly capture the pathology (11) requiring medical intervention.

The area requiring surgery will then be translated into a volumetric representation within the future hologram (38). This holographic volume, with precise location and dimensions, will serve as a guide for the practitioner during the procedure.

After completing Step A1, the images, typically formatted according to the DICOM protocol, are prepared for the next stage. In Step A2, these files are uploaded to a server, where they await processing on a dedicated computer for hologram creation.

### STEP B: Creation of the Hologram

The hologram is a representation of the medical images acquired during Step A. Its creation is performed in a separate environment from the patient scanning process and is typically handled by a user with a different level of expertise.

The process begins on a dedicated computer, where medical images are downloaded from a server (Step B1). The system can automatically identify key structures such as the skin, bones, and organs from the medical images, translating them into surfaces and volumes within the hologram (Step B2).

While the software used for hologram creation is not part of this patent, it is an integral component of the process. Without the hologram, the system cannot function.

Following segmentation (Step B2), the doctor for the patient reviews the medical images to identify the pathology (Step B3), which is then translated into a designated volume within the hologram. The system requires at least the segmentation of the skin, vital organs, and one target area for operation. Optionaly, an entry zone can have been defined by the doctor to avoid any contact with critical

Once these elements are identified, the hologram is generated (Step B4) and uploaded to a file storage system (Step B5), making it ready for use in Step C.

### STEP C: Medical Gesture

The system is designed for intuitive use, enabling operation by anyone. After identifying the target in Step B, the hologram containing all necessary data for the procedure is loaded into the system (C1). The 3D camera (31) then scans its field of view to locate a physical volume that matches the hologram's skin profile. This process (C2) is fully automated, utilizing the 3D camera (31) and the accompanying software on the computer (33). Matching the hologram with the real-world volume involves spatial comparison of the two. Once the camera identifies the matching volume (38), it continuously tracks the patient's body (10) to ensure stability. Additionally, the system can monitor the patient's breathing to confirm the body volume remains consistent with the one captured during Step A1. For example, the system detects differences in body volume between inspiration and expiration phases.

When the hologram is aligned, it is displayed on the screen (33), allowing the practitioner to activate operation mode. Optionally, the system highlights entry zones defined during Step B3, guiding the practitioner to restrict operations to safe zones and prevent the needle from contacting critical organs.

In operation mode, the practitioner uses the laser (22) to define the entry point (24) during Step C3. Optionally, the medical handle is equipped with a contact detector connected to the needle (23), enabling the system to recognize when the needle makes contact with the body (10). Upon contact, the system displays the target points that the needle must reach. The system determines the entry point using the laser, while the needle's angle is tracked via an IMU (Inertial Measurement Unit) embedded in the medical handle (21). Combining this data, the system calculates the needle's trajectory (36) and determines whether it aligns with the virtual target (37) during Step C4.

Once the correct angle is established, the practitioner can insert the needle into the real body (10) to reach the target (11). Optionally, the needle's progression within the body is monitored either through a telemeter embedded in the medical handle or via computer vision technology using the 3D camera.

### Part 4: Safety Features and Feedback Mechanisms

The system incorporates a range of advanced safety features and feedback mechanisms to ensure maximum precision, minimize risks, and enhance procedural outcomes. These elements are designed to provide real-time guidance and maintain patient safety throughout the procedure.

### 4.1. Real-Time Alignment and Monitoring

- The system uses a 3D camera (31) to continuously align the hologram with the patient's physical body (10). This ensures that any minor movement of the patient does not compromise the accuracy of the procedure.
- A fitness measurement algorithm dynamically adjusts the hologram alignment, preserving precise overlay during the intervention. This prevents misalignment that could result in incorrect needle placement.

### 4.2. Breathing Synchronization

- To account for changes in body volume due to respiration, the system monitors the patient's breathing cycle in real-time. This feature ensures that the body volume remains consistent with the hologram data captured during Step A1.
- Alerts are generated if discrepancies between inspiration and expiration volumes are detected, prompting the practitioner to adjust their actions accordingly.

### 4.3. Entry Zone Restriction

- The system highlights entry zones defined during Step B3, restricting the practitioner to operate only in predefined safe areas. This minimizes the risk of the needle contacting critical anatomical structures.
- If the practitioner attempts to penetrate outside these zones, the system issues a warning to prevent accidental injury.

### 4.4. Contact Detection and Feedback

- A contact detector integrated into the medical handle (21) identifies when the needle (23) touches the patient's body (10). This feedback is displayed on the system's screen, ensuring the practitioner is aware of the exact point of contact.
- The laser (22) and IMU provide additional feedback on the entry point and needle angle, enabling the system to compute an optimal trajectory and guide the practitioner with precision.

### 4.5. Collision Avoidance System

- By analyzing the needle's trajectory (36) in real-time, the system detects potential collisions with critical structures represented in the hologram. If a collision is likely, the system advises corrective actions to the practitioner before penetration.

### 4.6. Needle Progression Tracking

- The system monitors the needle's progression through optional telemeter sensors or by leveraging computer vision via the 3D camera. This ensures the needle reaches the intended depth and avoids unintended deviations.
- Feedback on the needle's distance to the target (11) is displayed on the screen, allowing the practitioner to make informed adjustments during the procedure.
- Additionally, an ultrasound probe can be used to provide real-time verification of the needle's position inside the body. This ensures accurate tracking of soft tissue structures, helps confirm needle penetration depth, and provides an extra safety layer by detecting potential deviations from the planned trajectory. The ultrasound probe can also verify that the target remains in the correct location and, if necessary, trigger a recalibration of the hologram to ensure accurate alignment with real anatomical structures.

### 4.7. Emergency Stop Mechanism

- In the event of a critical misalignment or unforeseen complication, the system includes an emergency stop mechanism. This halts the procedure and alerts the practitioner to reassess the situation, preventing further risk to the patient.

### 4.8. Intuitive Alerts and Warnings

- Visual and auditory alerts are integrated into the system to provide real-time feedback, such as alignment errors, trajectory deviations, or patient movement. This ensures the practitioner is continuously informed of the procedure's status and can act promptly.

These safety features and feedback mechanisms collectively establish a robust framework that enhances the reliability of the Medical Gesture system, ensuring safe and precise execution of minimally invasive procedures.

### Part 5: Applications and Advantages

The system is a groundbreaking innovation with versatile applications and distinct advantages that address critical challenges in modern medical procedures. This section outlines its key applications and the benefits it brings to healthcare professionals and patients alike.

### 5.1. Applications

### 5.1.1. Minimally Invasive Procedures

- **Biopsies:** The system ensures precise targeting of tissues for diagnostic sampling, reducing the risk of missing the intended area.
- **Targeted Injections:** Injections into specific tissues, such as nerve blocks or joint injections, are performed with enhanced accuracy.
- **Ablations and Treatments:** The system guides the practitioner in safely navigating to and treating areas affected by tumors or other pathologies.

### 5.1.2. Complex Surgical Procedures

- **Organ-Sensitive Interventions:** By highlighting critical anatomical structures, the system supports procedures near sensitive organs, minimizing accidental damage.
- **Emergency Interventions:** The portability of the system allows it to be deployed rapidly in emergency scenarios, facilitating quick and effective action.

### 5.1.3. Remote and Low-Resource Settings

- **Rural Healthcare:** The system's lightweight and portable design makes it ideal for remote locations where access to advanced medical equipment is limited.
- **Home-Based Care:** It enables certain interventions to be performed at a patient's residence, improving convenience and access for individuals with mobility challenges.

### 5.1.4. Training and Education

- The system's intuitive visual feedback and holographic guidance provide an excellent platform for teaching medical students and training practitioners in complex procedures. It simulates real-world scenarios, enhancing learning outcomes.

### 5.2. Advantages

### 5.2.1. Enhanced Precision and Safety

- By aligning holographic representations with the patient's body in real-time, the system minimizes errors in needle placement.
- Continuous monitoring of patient movement and breathing ensures interventions are performed with the highest level of accuracy.

### 5.2.2. Reduced Dependency on Expertise

- The system simplifies complex procedures, allowing general practitioners and less experienced medical staff to perform interventions with confidence. This democratizes access to advanced care.

### 5.2.3. Time Efficiency

- Automated alignment, trajectory calculation, and visual guidance reduce procedural time while maintaining high precision. This is particularly valuable in high-pressure or emergency environments.

### 5.2.4. Portability and Accessibility

- The lightweight design eliminates the need for dedicated infrastructure, making the system usable in a variety of settings, including outpatient clinics, rural facilities, and patient homes.

### 5.2.5. Cost-Effectiveness

- By replacing bulky, expensive electromagnetic or infrared tracking systems with a streamlined 3D camera and software-based approach, the system offers a more affordable solution without compromising quality.

### 5.2.6. Improved Patient Outcomes

- Enhanced precision reduces the risk of complications, ensures targeted treatments, and minimizes patient discomfort. These factors contribute to better recovery rates and overall satisfaction.

### 5.2.7. Scalability and Versatility

- The system can be adapted for use in various medical disciplines, from radiology and orthopedics to oncology and cardiology. Its flexibility ensures broad applicability across healthcare settings.

The innovation revolutionizes minimally invasive procedures, making them safer, more accessible, and more efficient. Its applications span diverse medical fields, while its advantages ensure a transformative impact on patient care and clinical practice worldwide.

## Claims

1. **A system for guiding medical instruments using three-dimensional (3D) imaging,** comprising:
∘ A 3D camera configured to capture real-time spatial data of a patient's body;
∘ A computer unit equipped with software to align a virtual hologram of the patient with the physical anatomy;
∘ A medical handle configured to hold a needle, incorporating:
▪ A laser for marking the entry point on the patient's body,
▪ An inertial measurement unit (IMU) to detect the angle of the needle, and
▪ A contact detector to identify when the needle touches the body;
∘ A feedback mechanism to display the needle's trajectory and target alignment on a screen;
∘ A monitoring system to track the patient's movement and breathing during the procedure.

2. **The system of claim 1,** wherein the computer unit dynamically adjusts the hologram alignment with the patient's body using a fitness measurement algorithm.

3. **The system of claim 1,** further comprising a telemetry module integrated into the medical handle to measure the depth of the needle within the body and display the remaining distance to the target.

4. **The system of claim 1,** wherein the hologram includes entry zones to restrict operations to predefined safe areas, preventing contact with critical anatomical structures.

5. **The system of claim 1,** wherein the monitoring system generates alerts in response to patient movement or misalignment between the hologram and the real anatomy.

6. **The system of claim 1,** further comprising a collision avoidance mechanism that detects potential trajectory conflicts with critical anatomical structures and issues corrective guidance to the practitioner.

7. **A method for guiding medical instruments during minimally invasive procedures,** comprising:
∘ Acquiring 3D medical images of the patient's body to generate a virtual hologram;
∘ Aligning the hologram with the patient's real anatomy using a 3D camera and a computer unit;
∘ Guiding the practitioner by displaying the hologram, needle trajectory, and target on a screen;
∘ Monitoring the patient's movement and breathing to ensure consistent alignment throughout the procedure;
∘ Providing visual and auditory feedback to assist the practitioner in adjusting the needle's position and angle.

8. **The method of claim 7,** wherein the hologram includes anatomical segmentation, highlighting critical structures and guiding the practitioner to avoid them during the procedure.

9. **The method of claim 7,** wherein the system tracks the needle's progression using either a telemeter integrated into the medical handle or computer vision technology with the 3D camera.

10. **The method of claim 7,** further comprising the ability to perform real-time trajectory calculations and display corrective suggestions if the needle deviates from the planned path.

11. **The method of claim 7,** wherein the system enables portability and remote operation, allowing medical interventions in rural or resource-limited settings.

12. **The system of claim 1 or the method of claim 7,** wherein the system is configured to operate autonomously, allowing non-specialist practitioners to perform complex procedures with minimal training.

13. **The system of claim 1 or the method of claim 7,** wherein an emergency stop mechanism halts the procedure in the event of critical misalignment or unforeseen complications.

14. **A medical guidance system,** as described in any of the preceding claims, wherein the system integrates laser-assisted targeting, computer vision-based trajectory monitoring, and real-time feedback to ensure safe and precise needle placement.

## Amended claims

### Amended claims in accordance with Rule 137(2) EPC.

1. **A system for guiding medical instruments using three-dimensional (3D) imaging, comprising:**
∘ A 3D camera (31) configured to capture spatial data of a patient's body (10);
∘ An electronic device attached to a clinical needle (23);
∘ A computer unit (33) equipped with software;
∘ A monitor configured to assist and guide the practitioner during the procedure;
**characterized in that the system further comprises:**
∘ A local optimization algorithm executed by the software to process spatial data from the 3D camera (31) and recognize the patient's body (10). The algorithm computes the most probable position of the patient's body (10) based on data captured by the 3D camera (31);
∘ A monitoring process executed by the software to overlay spatial data and a holographic model (38) of the patient (10) within a virtual scene referenced to the coordinate system of the 3D camera;
∘ An algorithm executed by the software to monitor the patient's breathing;
∘ An algorithm executed by the software to track the position and orientation of the medical instrument (23) within the same virtual scene as the holographic model (38) of the patient (10), all defined in the coordinate system of the 3D camera (31);
∘ An algorithm computing the trajectory (36) of the medical instrument (23) and detect collision with virtual target (37);
∘ A point laser module (22) integrated into the electronic device to mark the entry point (24) on the patient's body (10), the resulting red dot being detected by the 3D camera (31) and represented within the same virtual scene referenced to the camera's coordinate system;
∘ An Inertial Measurement Unit (IMU) integrated into the electronic device to measure the instrument (22)'s orientation;
∘ A contact detection module integrated into the electronic device to detect when the instrument touches the patient's body (10);
∘ A Graphical User Interface (GUI) running on the monitor, configured to display a virtual representation of the procedure with relevant guidance information;

2. **The system of claim 1,** wherein the computer unit (33) executes a local optimization algorithm that determines a transformation matrix aligning the holographic model (38) with the patient's body by minimizing the geometric error between anatomical landmarks extracted from the captured 3D data and corresponding features in the holographic model.

3. **The system of claim 1,** further comprising a telemetry module integrated into the medical handle (21) to measure the depth of the needle (23) within the patient's body (10) and display the remaining distance to the target (11).

4. **The system of claim 1,** wherein the hologram (38) includes entry zones (24) to restrict operations to predefined safe areas, preventing contact with critical anatomical structures such as bones or vital organs.

5. **The system of claim 1,** wherein the monitoring system generates alerts in response to patient (10) movement or misalignment between the hologram and the real anatomy, the misalignment being quantified by a score computed using a local optimization algorithm;

6. **The system of claim 1,** further comprising a collision avoidance mechanism that detects potential trajectory conflicts with critical anatomical structures and issues corrective guidance to the practitioner.

7. **The system of claim 1,** wherein an emergency stop mechanism is configured to interrupt the procedure in the event of a critical misalignment or unexpected complication, such as a sudden movement of the patient (10) during the procedure.

8. **The system of claim 1,** further comprising an algorithm executed by the software to guide the positioning of an ultrasound probe based on the spatial position and orientation of the clinical needle (23), so as to maintain optimal visibility of the needle trajectory (36) and ensure alignment with the target zone (37) during the procedure.
